# EUROPEAN PATENT SPECIFICATION

(11) **EP 4 129 157 B1**
(45) Date of publication and mention of the grant of the patent: **16.07.2025**
(21) Application number: 21780093.7
(22) Date of filing: 18.02.2021
(51) Int. Cl.: A61B 3/10, G02B 26/10

(54) **FUNDUS PHOTOGRAPHING DEVICE**
FOTOGRAFIEVORRICHTUNG FÜR AUGENHINTERGRUND
DISPOSITIF DE PHOTOGRAPHIE DE FOND D'OEIL

(30) Priority: 02.04.2020 JP 2020066916
(43) Date of publication of application: 08.02.2023
(73) Proprietor: QD Laser, Inc., Kanagawa 210-0855 (JP)
(72) Inventor: SUZUKI, Makoto, Kawasaki-shi, Kanagawa 210-0855 (JP); SAITO, Kazutaka, Kawasaki-shi, Kanagawa 210-0855 (JP); MORINO, Seiji, Kawasaki-shi, Kanagawa 210-0855 (JP)
(74) Representative: Haseltine Lake Kempner LLP
(86) International application number: PCT/JP2021/006103
(87) International publication number: WO 2021/199762

(56) References cited:
- WO-A1-2009/154134
- JP-A- 2016 510 628
- US-A1- 2012 257 166

## Description

### TECHNICAL FIELD

The present invention relates to a fundus photography device.

### BACKGROUND ART

There is known a scanning laser ophthalmoscope (SLO) that obtains a fundus image by scanning a light beam at high speed to irradiate the retina of a subject with the light beam and detecting reflected light from the retina with a light detector. Conventionally, a combination of a polygon mirror and a galvano mirror has been used for scanning a light beam. In addition, to reduce the size and cost, it has been proposed to use a micro electro mechanical system (MEMS) that biaxially drives to two-dimensionally scan a light beam instead of the polygon mirror and the galvano mirror (for example, Patent Document 1). Patent Document 2 discloses a portable MEMS-based scanning laser ophthalmoscope (MSLO).

### PRIOR ART DOCUMENT

### PATENT DOCUMENT

Patent Document 1: Japanese Patent Application Publication No. 2016-510628
Patent Document 2: United States Patent Application Publication No. US 2012/0257166 A

US 2012/257166 discloses a wide-field SLO for retinal imaging, comprising: laser illumination subassemblies 200 that generate optical illumination beams 210 for scanning the human eye 110, a 2D MEMS scanning minor 300 approximately 2 mm in diameter, a conic front objective 400, and a detector sub-assembly 500 detecting light scattered from the retina. 1

### SUMMARY OF THE INVENTION

### PROBLEM TO BE SOLVED BY THE INVENTION

However, it has been found that it is difficult to obtain a high-quality fundus image only by replacing the polygon mirror and the galvano mirror with a scanning unit such as a MEMS that biaxially drives.

The present invention has been made in view of above problems, and an object thereof is to provide a fundus photography device capable of obtaining a high-quality fundus image.

### MEANS FOR SOLVING THE PROBLEM

The present invention is a fundus photography device including: a light source; a scanning unit that includes a mirror that biaxially drives in a horizontal direction and a vertical direction to two-dimensionally scan a light beam emitted from the light source; an optical system that causes the light beam that has been reflected by the mirror of the scanning unit to enter an eye of a subject; and a light detector that detects the light beam that has been reflected by a retina of the subject, wherein an external diameter of the mirror of the scanning unit is 1.7 mm or greater and 2.0 mm or less.

In the above configuration, a configuration in which an optical magnification of the optical system is 0.8 times or greater and 1.2 times or less is employed.

In the above configuration, a configuration in which a resonant frequency in a horizontal direction of the scanning unit is 6 kHz or greater and 12 kHz or less is employed.

In the above configuration, a configuration in which a mechanical deflection angle in at least one of a horizontal direction or a vertical direction of the scanning unit is 13 degrees or greater and 16 degrees or less in half angle may be employed.

In the above configuration, a configuration in which the optical system includes an optical component that converts incident light beams, which have optical axes mutually diffusing and are diffusion lights, to light beams, which have optical axes mutually converging and are substantially parallel lights, and the light beam reflected by the mirror of the scanning unit is condensed between the scanning unit and the optical component, becomes diffusion light, enters the optical component, is converted to substantially parallel light by the optical component, and enters an eye of the subject may be employed.

In the above configuration, a configuration in which the optical system includes a first optical component and a second optical component, where the first optical component converts incident light beams, which have optical axes mutually diffusing and are substantially parallel lights, to light beams, which have optical axes parallel to each other and are convergent lights, and the second optical component converts the light beams emitted from the first optical component to light beams, which have optical axes mutually converging and are substantially parallel lights, and the light beam reflected by the mirror of the scanning unit enters the first optical component as substantially parallel light, is condensed between the first optical component and the second optical component, becomes diffusion light, enters the second optical component, is converted to substantially parallel light by the second optical component, and enters an eye of the subject may be employed.

In the above configuration, a configuration in which a signal processing unit that processes an output signal from the light detector is provided and an image generation unit that generates a fundus image of the subject, based on a signal processed by the signal processing unit may be employed.

### EFFECTS OF THE INVENTION

The present invention allows a high-quality fundus image to be obtained.

### BRIEF DESCRIPTION OF THE DRAWINGS

FIG. 1 is a block diagram of a fundus photography device in accordance with a first embodiment;
FIG. 2 illustrates an optical system of the fundus photography device in accordance with the first embodiment;
FIG. 3 is a diagram for describing scanning of a light beam;
FIG. 4A is a perspective view of a scanning unit, and FIG. 4B is an enlarged perspective view of the vicinity of a mirror;
FIG. 5 illustrates an optical system of a fundus photography device in accordance with a comparative example;
FIG. 6 illustrates a relationship between the diameter of the light beam at the time of entering a cornea and the diameter of the light beam on a retina;
FIG. 7 illustrates a relationship between the external diameter of the mirror of the scanning unit and the horizontal resonant frequency of the scanning unit;
FIG. 8A to FIG. 8D present simulation results of the deformation of the mirror of the scanning unit;
FIG. 9 illustrates an optical system of a fundus photography device in accordance with a second embodiment; and
FIG. 10 is an optical system of a fundus photography device in accordance with a third embodiment.

### MODES FOR CARRYING OUT THE INVENTION

Hereinafter, with reference to the drawings, embodiments of the present invention will be described.

### First Embodiment

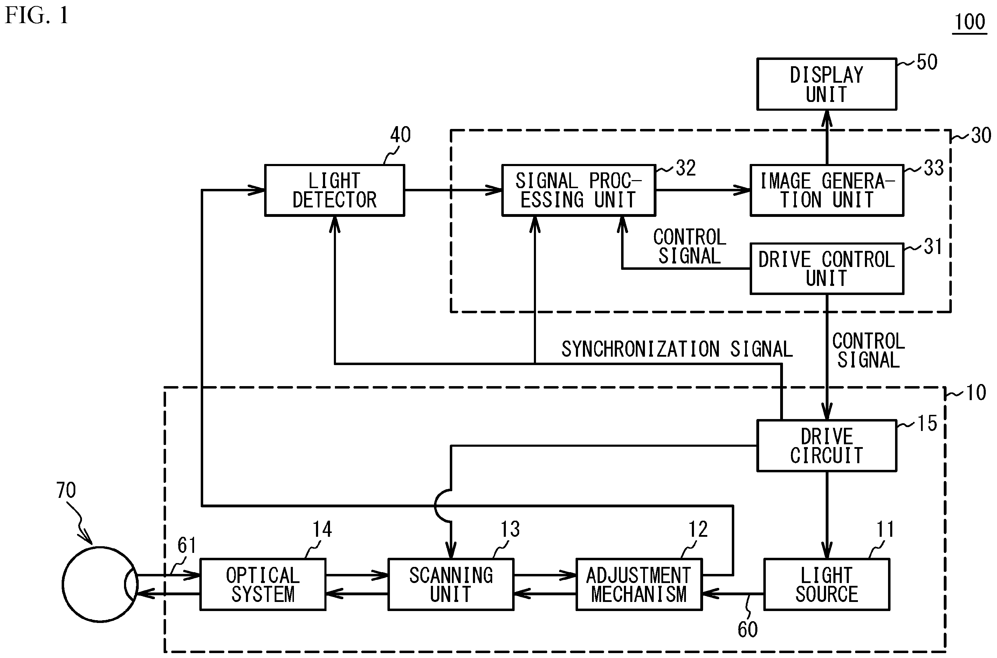

FIG. 1 is a block diagram of a fundus photography device in accordance with a first embodiment. As illustrated in FIG. 1, a fundus photography device 100 includes a projection unit 10, a control unit 30, a light detector 40, and a display unit 50. The projection unit 10 includes a light source 11, an adjustment mechanism 12, a scanning unit 13, an optical system 14, and a drive circuit 15. The control unit 30 includes a drive control unit 31, a signal processing unit 32, and an image generation unit 33.

The drive control unit 31 generates a control signal for controlling the light beam with which the retina of a subject is irradiated. The drive circuit 15 drives the light source 11 and the scanning unit 13 on the basis of the control signal of the drive control unit 31.

The light source 11 emits an invisible light beam of infrared laser light having a wavelength of about 785 nm to 1.4 µm, for example. The light source 11 may emit a visible light beam of, for example, a red laser light (wavelength: about 610 nm to 660 nm), a green laser light (wavelength: about 515 nm to 540 nm), and/or a blue laser light (wavelength: about 440 nm to 480 nm).

The adjustment mechanism 12 includes a collimating lens, a toric lens, an aperture, and/or the like, and shapes a light beam 60 emitted by the light source 11. The light beam 60 is, for example, an infrared laser light, a red laser light, a green laser light, or a blue laser light.

The scanning unit 13 is a scanner having a mirror that biaxially swings and drives to two-dimensionally scan the light beam 60. The light beam 60 is scanned by the scanning unit 13 in the horizontal direction (a main scanning direction) and the vertical direction (a sub-scanning direction). The scanning unit 13 is, for example, a micro electro mechanical system (MEMS).

The optical system 14 irradiates an eye 70 of the subject with the light beam 60 that has been scanned by being reflected by the mirror of the scanning unit 13.

The light detector 40 is a photodetector such as, for example, an avalanche photodiode, and detects a reflected light 61 that has been reflected by the eye 70 of the subject and has passed through the optical system 14, the scanning unit 13, and the adjustment mechanism 12. The light detector 40 starts the detection at a timing when the light source 11 emits the light beam 60 on the basis of the synchronization signal from the drive circuit 15.

The signal processing unit 32 processes the output signal of the light detector 40, on the basis of the control signal from the drive control unit 31. The signal processing unit 32 starts the process at a timing when the light source 11 emits the light beam 60 on the basis of the synchronization signal from the drive circuit 15.

The image generation unit 33 generates a fundus image on the basis of the signal processed by the signal processing unit 32. The display unit 50 is, for example, a liquid crystal display, and displays a fundus image generated by the image generation unit 33.

The drive control unit 31, the signal processing unit 32, and the image generation unit 33 may be implemented by a processor such as a central processing unit (CPU) in cooperation with a program. The drive control unit 31, the signal processing unit 32, and the image generation unit 33 may be circuits that are specially designed. The drive control unit 31, the signal processing unit 32, and the image generation unit 33 may be one circuit or different circuits.

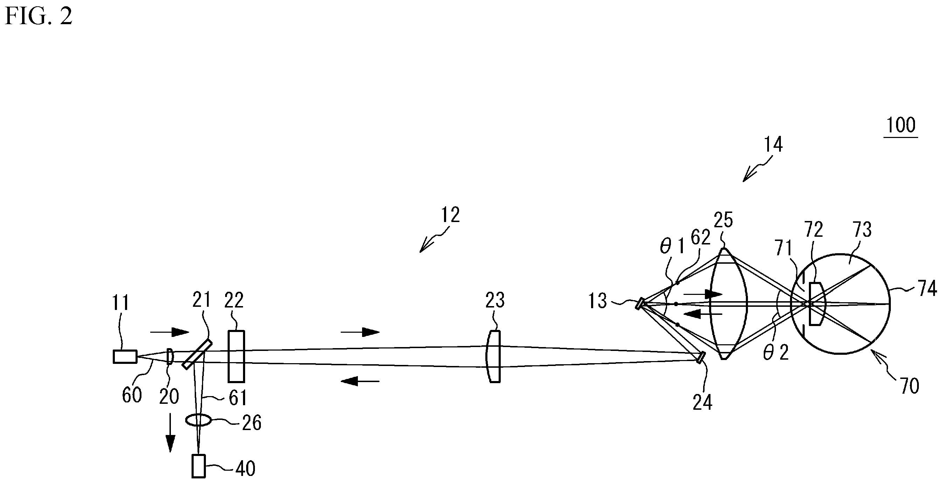

FIG. 2 illustrates the optical system of the fundus photography device in accordance with the first embodiment. As illustrated in FIG. 2, the light beam 60 emitted by the light source 11 is converted from diffusion light to substantially parallel light by a collimating lens 20, and then passes through a half mirror 21, a diopter adjustment lens 22, and a diopter adjustment lens 23 to enter a reflection mirror 24. The light beam 60 enters the reflection mirror 24 as convergent light. The light beam 60 is reflected by the reflection mirror 24 to enter the scanning unit 13, and is two-dimensionally scanned by being reflected by the scanning unit 13.

The light beam 60 that has been two-dimensionally scanned by being reflected by the scanning unit 13 passes through a projection lens 25 and enters the eye 70 of the subject. The optical axes of the light beams 60 scanned by the scanning unit 13 mutually diffuse, and each of the light beams 60 is convergent light until it reaches a focal point 62. Each light beam 60 enters the projection lens 25 as diffusion light. The projection lens 25 converts the light beams 60 that are reflected in different directions by the scanning unit 13 and have optical axes mutually diffusing, to the light beams 60 having optical axes mutually converging, and converts each of the light beams 60 from diffusion light to substantially parallel light and causes them to enter the eye 70 of the subject. The light beam 60 passes through a pupil 71, converges in a crystalline lens 72 or near the crystalline lens 72, passes through a vitreous body 73, and focuses substantially at a retina 74. That is, the retina 74 of the subject is irradiated with the light beam 60 using Maxwellian view. The term "substantially parallel" means that the light beam 60 is substantially parallel to such an extent that the light beam 60 can be focused substantially at the retina 74 (the same applies to the following description). The projection lens 25 is a convex lens and has an optical magnification of 0.8 times or greater and 1.2 times or less, which will be described later. The optical magnification is a value represented by, for example, a ratio of a distance between the projection lens 25 and the mirror of the scanning unit 13 to a distance between the projection lens 25 and the crystalline lens 72 of the subject or a convergence position of the light beam 60 near the crystalline lens 72.

The light beam 60 is reflected by the retina 74 of the subject. The reflected light 61 reflected by the retina 74 travels back along the optical path along which the light beam 60 has advanced toward the retina 74, in the order of the projection lens 25, the scanning unit 13, the reflection mirror 24, the diopter adjustment lens 23, and the diopter adjustment lens 22, is reflected by the half mirror 21, passes through a condenser lens 26, and enters the light detector 40. The light detector 40 detects the reflected light 61 reflected by the retina 74. The image generation unit 33 generates a fundus image on the basis of the signal obtained by processing a detection result such as the luminance variation of the reflected light 61 by the light detector 40 by the signal processing unit 32.

The collimating lens 20, the diopter adjustment lens 22, and the diopter adjustment lens 23 correspond to the adjustment mechanism 12 in FIG. 1. The projection lens 25 corresponds to the optical system 14 in FIG. 1.

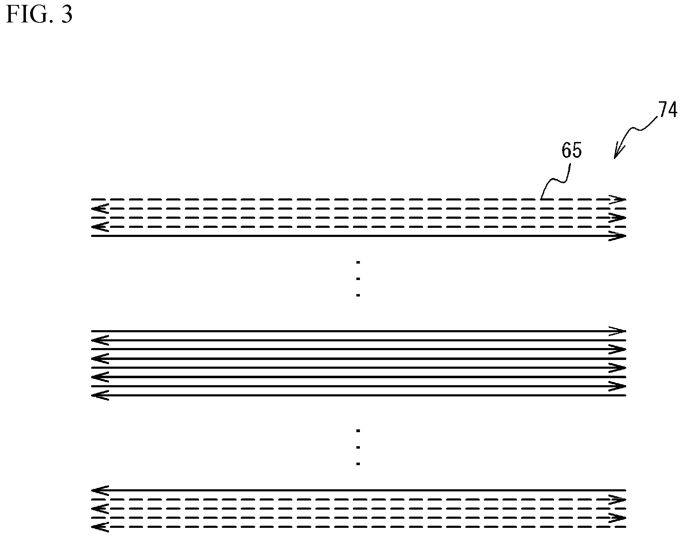

FIG. 3 is a diagram for describing scanning of the light beam. As illustrated in FIG. 3, the scanning unit 13 performs raster scanning with the light beam 60 from the upper left to the lower right as indicated by arrows 65. In raster scanning, the horizontal direction is the main scanning direction, and the vertical direction is the sub-scanning direction. For example, the number of scanning lines is 640. The retina 74 is not irradiated with the light beam 60 unless the light source 11 emits the light beam 60 even when the mirror of the scanning unit 13 biaxially swings and drives. For example, in the dotted arrows 65 in FIG. 3, the light beam 60 is not emitted. The drive circuit 15 synchronizes the emission of the light beam 60 from the light source 11 and the biaxial swing drive of the scanning unit 13. This causes the light source 11 to emit the light beam 60 in the solid line arrows 65.

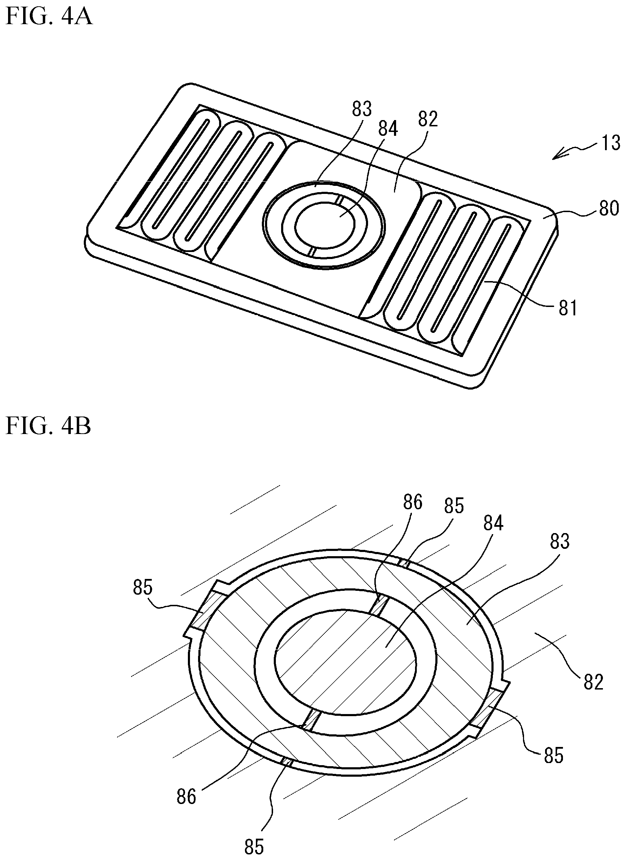

FIG. 4A is a perspective view of the scanning unit, and FIG. 4B is an enlarged perspective view of the vicinity of the mirror of the scanning unit. As illustrated in FIG. 4A and FIG. 4B, the scanning unit 13 is, for example, a MEMS, and includes an outer frame 80, a suspension 81, an inner frame 82, a piezoelectric unit 83, and a mirror 84. The inner frame 82 is fixed to the outer frame 80 through the suspension 81. The piezoelectric unit 83 has a structure in which a piezoelectric film such as a PZT film is sandwiched between upper and lower electrodes, and is fixed to the inner frame 82 by four first torsion bars 85 arranged at 90-degree intervals. The mirror 84 is fixed to the piezoelectric unit 83 by two second torsion bars 86 arranged at 180-degree intervals.

In the scanning unit 13, warpage behavior caused by applying a voltage to the piezoelectric unit 83 is transmitted to the mirror 84 through the second torsion bar 86, so that the mirror 84 biaxially swings and drives. Accordingly, when the light beam 60 enters the mirror 84, the light beam 60 is scanned in two-dimensional directions. In FIG. 4A and FIG. 4B, the piezoelectric MEMS is illustrated as an example, but the scanning unit 13 may be a capacitive MEMS or other elements.

The outer peripheral region of the mirror 84 is curved (sagged) for manufacturing reasons. Therefore, when the light beam 60 is reflected by the entire region including the outer peripheral region of the mirror 84, it is difficult to reflect the light beam 60 only in a desired direction. Therefore, to reflect the light beam 60 satisfactory, the light beam 60 is made to be reflected by a region further inward than the outer peripheral region where the mirror 84 curves. That is, the region excluding the outer peripheral region where curvature occurs of the mirror 84 is an effective region capable of reflecting the light beam 60 satisfactory. Therefore, the effective diameter, which is the length of the effective region of the mirror 84, is equal to the value obtained by subtracting the length of the outer peripheral region where curvature occurs from the length of the mirror 84. For example, in the case that the mirror 84 is circular and curvature occurs in the area within 0.1 mm from the edge of the mirror 84, the effective diameter of the mirror 84 is equal to the value obtained by subtracting 0.2 mm from the diameter, which is the external diameter of the mirror 84. **In** addition, in the case that the mirror 84 is elliptic, the effective diameter of the mirror 84 is equal to the value obtained by subtracting the length of the outer peripheral region where curvature occurs from the minor diameter, which is the external diameter. The external diameter of the mirror 84 is 1.7 mm or greater and 2.0 mm or less, and the effective diameter is 1.5 mm or greater and 1.8 mm or less, which will be described later.

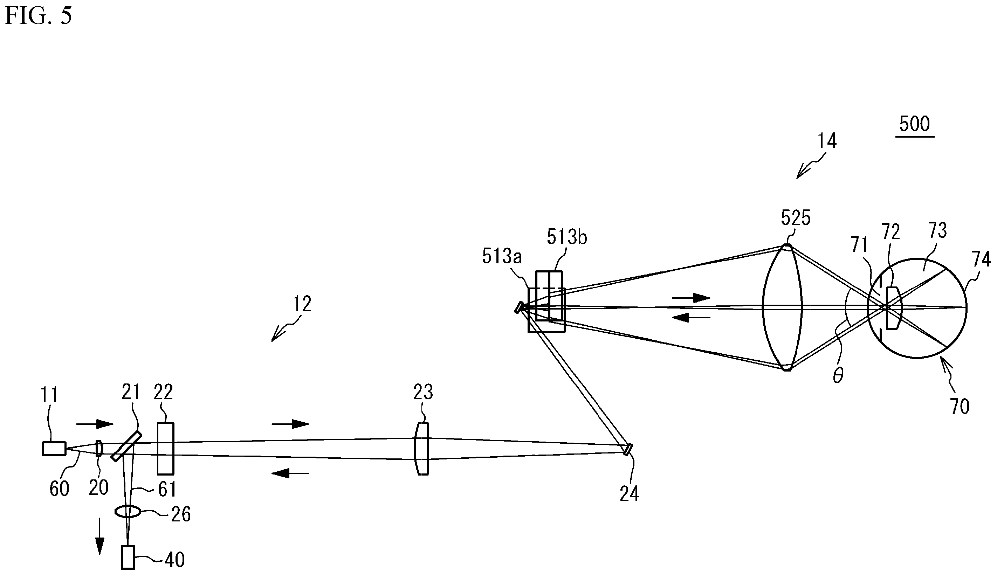

FIG. 5 is an optical system of a fundus photography device in accordance with a comparative example. As illustrated in FIG. 5, in a fundus photography device 500 of the comparative example, the light beam 60 is two-dimensionally scanned using a combination of a polygon mirror 513a and a galvano mirror 513b. The polygon mirror 513a scans the light beam 60 in the horizontal direction (the main scanning direction), for example, and the galvano mirror 513b scans the light beam 60 in the vertical direction (the sub-scanning direction), for example. **In** the fundus photography device 500, the optical magnification of the projection lens 525 is 2.5 times. The optical magnification is a value represented by a ratio of the distances between the projection lens 525 and the polygon mirror 513a and the galvano mirror 513b to the distance between the projection lens 525 and the crystalline lens 72 of the subject or a convergence position of the light beam 60 near the crystalline lens 72. Other structures are the same as those of the fundus photography device 100 of the first embodiment, and the description thereof is thus omitted.

In the fundus photography device 500 of the comparative example, the optical magnification of the projection lens 525 is 2.5 times for the following reason. When a fundus image is obtained by irradiating the retina 74 with the light beam 60, a viewing angle of 60 degrees or greater in full angle is required to find a peripheral disease part. That is, the angle θ at which the light beams 60 converge at the convergence point in the eye 70 is required to be 60 degrees or greater. However, when the polygon mirror 513a and the galvano mirror 513b are used, it is difficult particularly for the galvano mirror 513b to scan the light beam 60 at a wide angle. For this reason, in the fundus photography device 500 of the comparative example using the polygon mirror 513a and the galvano mirror 513b, the optical magnification of the projection lens 525 is set to 2.5 times so that the viewing angle at the time of obtaining a fundus image is about 60 degrees.

The fundus photography device 500 using the polygon mirror 513a and the galvano mirror 513b is large and expensive. Therefore, instead of the polygon mirror 513a and the galvano mirror 513b, it is conceivable to use the scanning unit 13 that biaxially swings and drives to biaxially scan the light beam 60. However, the inventor has found that it is difficult to obtain a high-quality fundus image only by arranging the scanning unit 13 at the positions of the polygon mirror 513a and the galvano mirror 513b. This will be described below.

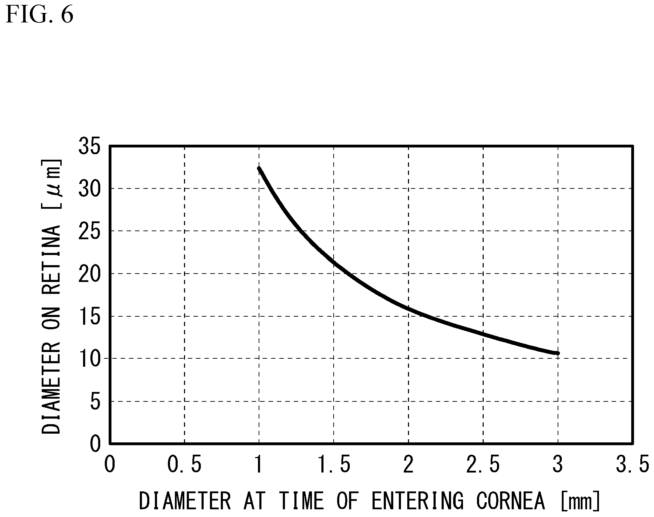

To ensure the resolutions on the retina and the SN ratio of the fundus image, the fundus photography device is required to irradiate the entire width of approximately 11 mm on the retina corresponding to a viewing angle of 45 degrees (full angle) with about 450 light beams 60 without interference. That is, the spot size (the diameter) of the light beam 60 on the retina 74 is required to be 25 µm or less. FIG. 6 illustrates a relationship between the diameter of the light beam at the time of entering the cornea and the diameter of the light beam on the retina. As illustrated in FIG. 6, as the diameter of the light beam 60 at the time of entering the cornea increases, the diameter of the light beam 60 on the retina 74 decreases. It is revealed that the diameter (the spot size) of the light beam 60 on the retina 74 can be made to be 25 µm or less by adjusting the diameter of the light beam 60 at the time of entering the cornea to be 1.25 mm or greater. The reason why the diameter of the light beam 60 on the retina 74 decreases as the diameter of the light beam 60 at the time of entering the cornea increases is as follows. The crystalline lens 72 exists on the retina side of the cornea, and the cornea and the crystalline lens 72 have optical characteristics having a positive light condensing power as a convex lens. When the diameter of the light beam 60 incident on it is large, the light condensing power becomes large and the spot size of the retina 74 becomes small. By contrast, when the diameter of the incident light beam 60 is small, the light condensing power is small and it is difficult to condense the light beam 60, and the spot size of the retina 74 does not become sufficiently small.

In the fundus photography device 500 of the comparative example, the optical magnification of the projection lens 525 is 2.5 times. Therefore, when the scanning unit 13 is arranged at the positions of the polygon mirror 513a and the galvano mirror 513b without change, it is required to adjust the effective diameter of the mirror 84 of the scanning unit 13 to be 3.2 mm or greater to make the diameter of the light beam 60 at the time of entering the cornea 1.25 mm or greater. However, it is not practical to adjust the effective diameter of the mirror 84 of the scanning unit 13 to be 3.2 mm or greater. This is because of the following reasons.

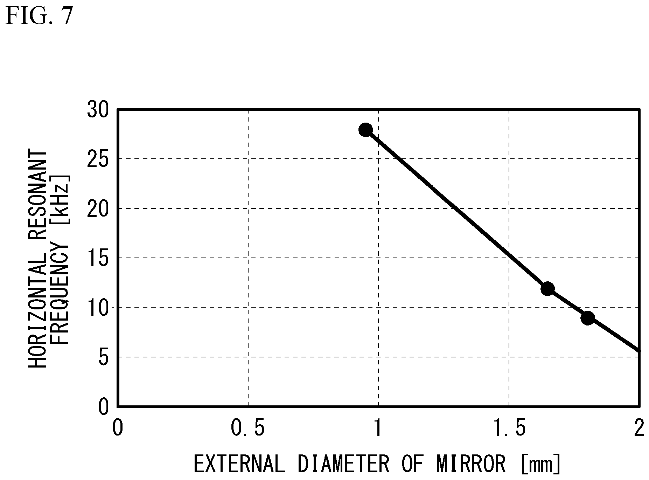

FIG. 7 illustrates a relationship between the external diameter of the mirror of the scanning unit and the horizontal resonant frequency of the scanning unit. As illustrated in FIG. 7, as the external diameter of the mirror 84 of the scanning unit 13 increases, the horizontal resonant frequency of the scanning unit 13 decreases. This is because as the external diameter of the mirror 84 of the scanning unit 13 increases, the inertia increases and the deformation of the mirror 84 increases, and thus, the horizontal resonant frequency cannot be increased. In the case that the difference between the external diameter and the effective diameter of the mirror 84 is 0.2 mm, the horizontal resonant frequency can be about 12 kHz when the external diameter of the mirror 84 is 1.6 mm, can be about 10 kHz when 1.7 mm, can be about 9 kHz when 1.8 mm, and can be about 6 kHz when 2.0 mm. Here, under the condition that the scanning unit 13 is a circular MEMS, the value of the difference between the external diameter and the effective diameter of the mirror 84 is adjusted to be 0.2 mm, but this value may differ depending on the characteristics of the MEMS.

When a fundus image is obtained by irradiating the retina 74 with the light beam 60 by raster scanning, it is required to set the number of scanning lines to 640 or greater and a frame rate to 15 fps or greater to obtain a high-quality fundus image. To achieve a frame rate of 15 fps in raster scanning with 640 scanning lines, the horizontal resonant frequency of the scanning unit 13 is required to be 6 kHz or greater. From FIG. 7, to set the horizontal resonant frequency of the scanning unit 13 to 6 kHz or greater, the external diameter of the mirror 84 of the scanning unit 13 is required to be 2.0 mm or less (1.8 mm or less as the effective diameter of the mirror 84). For this reason, it is not practical to adjust the effective diameter of the mirror 84 of the scanning unit 13 to be 3.2 mm or greater to obtain the resonant frequencies required in the fundus photography device.

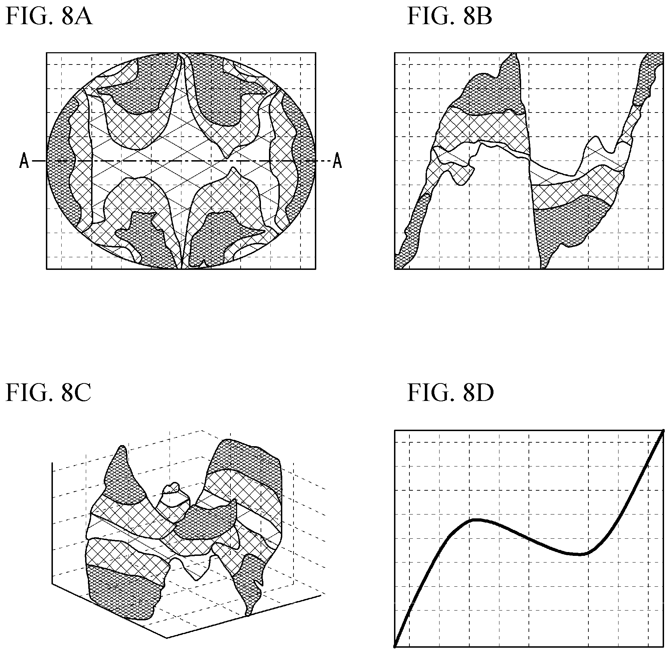

The deformation of the mirror 84 of the scanning unit 13 was simulated for the case in which the external diameter of the mirror 84 of the scanning unit 13 is 1.6 mm, the mechanical deflection angle is 14.6 degrees in half angle, and the horizontal resonant frequency is 12 kHz. FIG. 8A to FIG. 8D present simulation results of the deformation of the mirror of the scanning unit. FIG. 8A illustrates the mirror 84 as viewed from above, FIG. 8B illustrates the mirror 84 as viewed from the front, and FIG. 8C illustrates the mirror 84 as viewed from an angle. FIG. 8D is a graph presenting the deformation along line A-A in FIG. 8A. In FIG. 8A to FIG. 8C, a part in which the deformation amount is small is indicated by coarse hatching, and a part in which the deformation amount in the positive direction and the negative direction is large is indicated by dense hatching.

As presented in FIG. 8A to FIG. 8D, in the case that the external diameter of the mirror 84 was 1.6 mm, the mechanical deflection angle was 14.6 degrees, and the horizontal resonant frequency was 12 kHz, a high-order deformation mode was generated in the mirror 84, and the largest deformation amount was about ±100 nm. It is considered that such a high-order deformation mode becomes larger as the external diameter of the mirror 84 becomes larger. Therefore, when the effective diameter of the mirror 84 is adjusted to be 3.2 mm or greater, it is considered that the high-order deformation mode generated in the mirror 84 interferes with the optical performance, and it is not practical to adjust the effective diameter of the mirror 84 to be 3.2 mm or greater. In addition, in the case that the effective diameter of the mirror 84 is adjusted to be 3.2 mm or greater, it is considered that a large deflection angle cannot be obtained because of an increase in air resistance, and in this respect, it is not practical to adjust the effective diameter of the mirror 84 to 3.2 mm or greater.

In the case that the scanning unit 13 that biaxially swings and drives to two-dimensionally scan the light beam 60 is used as in the fundus photography device 100 of the first embodiment, the scanning angle θ1 (see FIG. 2) of the light beam 60 can be made to be large compared with that in the case in which the polygon mirror 513a and the galvano mirror 513b are used. In this case, even when the optical magnification of the projection lens 25 is set to 0.8 times or greater and 1.2 times or less, the convergence angle θ2 (see FIG. 2) of the light beam 60 at the convergence point in the eye 70 becomes large, and it is possible to achieve that the viewing angle at the time of obtaining the fundus image becomes about 60 degrees. In this case, the spot size of the light beam 60 on the retina 74 can be made to be 25 µm or less by adjusting the effective diameter of the mirror 84 of the scanning unit 13 to be 1.5 mm or greater (adjusting the external diameter to be 1.7 mm or greater).

In the first embodiment, the external diameter of the mirror 84 of the scanning unit 13 is 1.7 mm or greater and 2.0 mm or less. When the external diameter of the mirror 84 is 1.7 mm or greater (the effective diameter is, for example, 1.5 mm or greater), the spot size of the light beam 60 on the retina 74 can be made to be 25 µm or less, and the resolutions on the retina 74 and the SN ratio of the fundus image can be ensured. By adjusting the external diameter of the mirror 84 to be 2.0 mm or less (the effective diameter is, for example, 1.8 mm or less), raster scanning with 640 or more scanning lines and a frame rate of 15fps or greater can be performed. Thus, a high-quality fundus image can be obtained. In addition, since the diameter of the light beam 60 at the time of entering the cornea becomes 2.5 mm or less, even non-mydriasis is hardly blocked by the iris because the diameter of the human pupil is generally about 2 mm to 8 mm, and a fundus image can be obtained. The external diameter of the mirror 84 may be 1.8 mm or greater and 2.0 mm or less, may be 1.7 mm or greater and 1.9 mm or less, or may be 1.8 mm or greater and 1.9 mm or less. That is, the effective diameter of the mirror 84 may be 1.6 mm or greater and 1.8 mm or less, may be 1.5 mm or greater and 1.7 mm or less, or may be 1.6 mm or greater and 1.7 mm or less.

Since the scanning unit 13 can increase the scanning angle θ1, even when the optical magnification of the projection lens 25 (the optical system) is adjusted to be 0.8 times or greater and 1.2 times or less, it is possible to achieve that the viewing angle at the time of obtaining the fundus image is about 60 degrees. In this case, by adjusting the external diameter of the mirror 84 of the scanning unit 13 to be 1.7 mm or greater (adjusting the effective diameter to be, for example, 1.5 mm or greater), the spot size of the light beam 60 of the retina 74 can be made to be 25 µm or less. The optical magnification of the projection lens 25 (the optical system) may be 0.8 times or greater and 1.1 times or less, may be 0.9 times or greater and 1.2 times or less, and may be 0.9 times or greater and 1.1 times or less, and may be 1.0 time.

When a fundus image is obtained by irradiating the retina 74 with the light beam 60 that is two-dimensionally scanned by the scanning unit 13, to obtain a high-quality fundus image, the resonant frequency in the horizontal direction (the resonant frequency in the main scanning direction) of the mirror 84 of the scanning unit 13 is preferably 6 kHz or greater, more preferably 7 kHz or greater, further preferably 8 kHz or greater. To prevent the high-order deformation mode generated in the mirror 84 from increasing and interfering with the optical performance, the resonant frequency in the horizontal direction of the mirror 84 of the scanning unit 13 is preferably 12 kHz or less, more preferably 10 kHz or less, further preferably 9 kHz or less.

To achieve a viewing angle of about 60 degrees at the time of obtaining a fundus image, the mechanical deflection angle in either one of the horizontal direction and the vertical direction, preferably the mechanical deflection angles in both the horizontal direction and the vertical direction of the mirror 84 of the scanning unit 13 are preferably 13 degrees or greater in half angle, more preferably 13.5 degrees or greater in half angle, further preferably 14 degrees or greater in half angle. In consideration of air resistance, the deformation of the mirror 84, and the like, the mechanical deflection angle in either one of the horizontal direction and the vertical direction, preferably the mechanical deflection angles in both the horizontal direction and the vertical direction of the mirror 84 of the scanning unit 13 are preferably 16 degrees or less in half angle, more preferably 15.5 degrees or less in half angle, further preferably 15 degrees or less in half angle.

The projection lens 25 (an optical component) converts the light beams 60 that are reflected in different directions by the scanning unit 13 and have optical axes mutually diffusing, to the light beams 60 having optical axes mutually converging, and converts each light beam 60 from diffusion light to substantially parallel light. The light beam 60 is condensed between the scanning unit 13 and the projection lens 25, then becomes diffusion light, enters the projection lens 25, is converted to substantially parallel light by the projection lens 25, and enters the eye 70 of the subject. Use of such a projection lens 25 to constitute the optical system 14 allows the fundus photography device 100 to be miniaturized.

### Second Embodiment

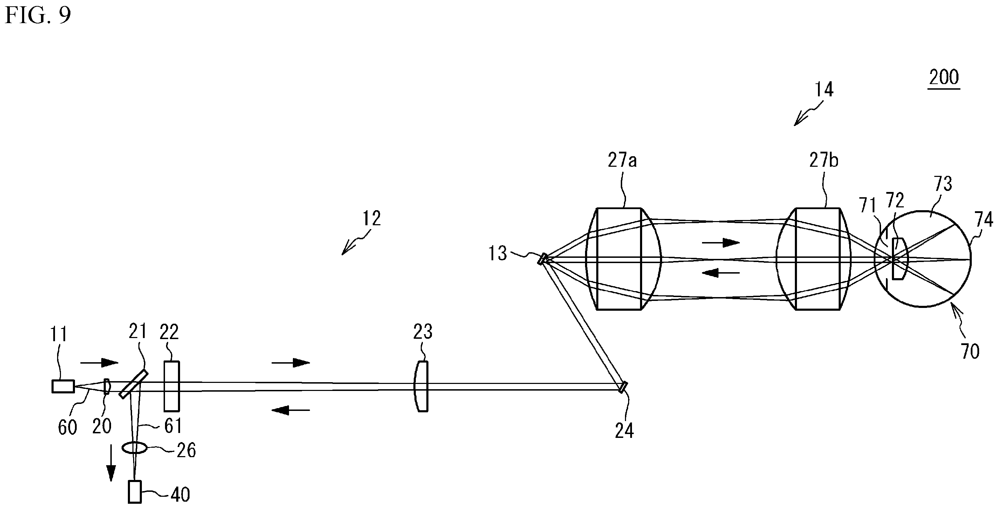

FIG. 9 illustrates an optical system of a fundus photography device in accordance with a second embodiment. As illustrated in FIG. 9, in a fundus photography device 200 of the second embodiment, the optical system 14 that irradiates the eye 70 of the subject with the light beam 60 reflected by the scanning unit 13 includes a projection lens 27a (a first optical component) and a projection lens 27b (a second optical component). The projection lens 27a converts the light beams 60 that are reflected in different directions by the scanning unit 13 and have optical axes mutually diffusing, to the light beams 60 having optical axes substantially parallel to each other, and converts each light beam 60 from substantially parallel light to convergent light. That is, the light beams 60 that have been reflected in different directions by the scanning unit 13 becomes parallel to each other by the projection lens 27a. The projection lens 27b converts the light beams 60 emitted from the projection lens 27a to the light beams 60 having optical axes mutually converging, and converts each light beam 60 from diffusion light to substantially parallel light. The light beam 60 enters the projection lens 27a as substantially parallel light, is condensed between the projection lens 27a and the projection lens 27b, then becomes diffusion light, enters the projection lens 27b, is converted to substantially parallel light by the projection lens 27b, and enters the eye 70 of the subject. Other configurations are the same as those of the fundus photography device 100 of the first embodiment, and the description thereof is thus omitted.

In the case that the optical system 14 includes the projection lens 27a (a first optical component) and the projection lens 27b (a second optical component) as in the fundus photography device 200 of the second embodiment, it is easy to achieve an optical magnification of 0.8 times or greater and 1.2 times or less for the optical system 14.

### Third Embodiment

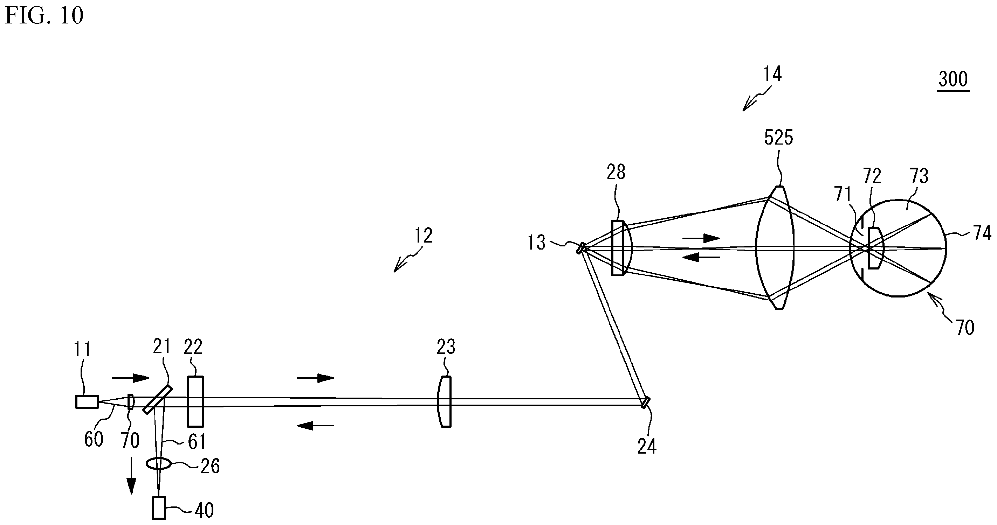

accordance with a third embodiment. As illustrated in FIG. 10, in a fundus photography device 300 of the third embodiment, the optical system 14 that irradiates the eye 70 of the subject with the light beam 60 reflected by the scanning unit 13 includes a converter lens 28 between the scanning unit 13 and a projection lens 525, in addition to the projection lens 525, and uses the scanning unit 13 that is a MEMS instead of the polygon mirror 513a and the galvano mirror 513b in FIG. 5. Other configurations are the same as those of the fundus photography device 500 of the comparative example, and the description thereof is thus omitted.

In the configuration of the third embodiment, by providing the converter lens 28 between the scanning unit 13 and the projection lens 525, even when the polygon mirror 513a and the galvano mirror 513b are replaced with the scanning unit 13 that is a MEMS, the fundus photography device can be structured without replacing the projection lens 525. The projection lens 525 is the projection lens used in the comparative example of FIG. 5, and is used in scanning by the polygon mirror and the galvano mirror.

In the first to third embodiments, the case in which a MEMS is used as the scanning unit 13 that biaxially drives to two-dimensionally scan the light beam 60 has been described as an example, but the scanning unit 13 may be other than the MEMS.

Although preferred embodiments of the present invention have been described so far, the present invention is not limited to those particular embodiments, and various changes and modifications may be made to them within the scope of the invention claimed herein.

## Claims

1. A fundus photography device comprising:
a light source (1);
a scanning unit (13) that includes a mirror configured to biaxially drive in a horizontal direction and a vertical direction to two-dimensionally scan a light beam (60) emitted from the light source;
an optical system (14) configured to cause the light beam that has been reflected by the mirror of the scanning unit to enter an eye (70) of a subject; and
a light detector (40) configured to detect the light beam that has been reflected by a retina (74) of the subject,
wherein an external diameter of the mirror of the scanning unit is 1.7 mm or greater and 2.0 mm or less, wherein the mirror of the scanning unit is circular or elliptic, and wherein the external diameter is a diameter of the mirror of the scanning unit when the mirror of the scanning unit is circular, and is a minor diameter of the mirror of the scanning unit when the mirror of the scanning unit is elliptic,
wherein an effective diameter of the mirror of the scanning unit is 1.5 mm or greater and 1.8mm or less wherein the effective diameter of the mirror of the scanning unit is equal to a value obtained by subtracting 0.2mm from the external diameter of the mirror of the scanning unit,
wherein an optical magnification of the optical system is 0.8 times or greater and 1.2 times or less,
wherein a resonant frequency in the horizontal direction of the scanning unit is 6 kHz or greater and 12 kHz or less.

2. The fundus photography device according to claim 1, wherein a mechanical deflection angle in at least one of the horizontal direction or the vertical direction of the scanning unit (13) is 13 degrees or greater and 16 degrees or less in half angle.

3. The fundus photography device according to any one of claims 1 to 2, wherein a diameter of the light beam at the time of entering the eye (70) of the subject is 1.25 mm or greater, and a spot size of the light beam on the retina (74) is 25 µm or less.

4. The fundus photography device according to any one of claims 1 to 3, further comprising:
a signal processing unit (32) configured to process an output signal from the light detector (40); and
an image generation unit (33) configured to generate a fundus image of the subject, based on a signal processed by the signal processing unit.

5. The fundus photography device according to any of claims 1 to 4, further comprising:
An adjustment mechanism (12) that includes a collimating lens (20) and a diopter adjustment lens (23),
Wherein the scanning unit (13) two-dimensionally scan the light beam (60) that has been emitted from the light source (11), converted to substantially parallel light by the collimating lens (20) and then converted to convergent light by the diopter adjustment lens (23),
Wherein the optical system (14) includes a projection lens (25) that causes the light beam that has been reflected by the mirror of the scanning unit to enter the eye (70) of the subject,
wherein the light beam that has been converted to the convergent light is reflected in different directions by the mirror of the scanning unit,
wherein a plurality of the light beams reflected in the different directions are converged in a crystalline lens (72) of the subject or near the crystalline lens by the projection lens (25) and
wherein each of the plurality of the light beams reflected in the different directions is condensed between the scanning unit and the projection lens (25), is then diffused, is converted to substantially parallel light by the projection lens (25), and enters the eye of the subject.

6. The fundus photography device according to any of claims 1 to 4, further comprising:
An adjustment mechanism (12) that includes a collimating lens (20),
Wherein the scanning unit (13) two-dimensionally scans the light beam (60) that has been emitted from the light source (11) and converted to substantially parallel light by the collimating lens (20),
wherein the optical system (14) includes a first projection lens (27a) and a second projection lens (27b),
wherein the light beam that has been converted to the substantially parallel light by the collimating lens (20) is reflected in different directions by the mirror of the scanning unit,
wherein a plurality of the light beams reflected in the different directions are caused to be parallel to each other by the first projection lens (27a), enter the second projection lens (27b), and are caused to be converged in a crystalline lens (72) of the subject or near the crystalline lens by the second projection lens (27b), and
wherein each of the plurality of the light beams reflected in the different directions is converted to convergent light by the first projection lens (27a), is condensed between the first projection lens (27a) and the second projection lens (27b), is then diffused, is converted to substantially parallel light by the second projection lens (27b), and enters the eye of the subject.

## Patentansprüche

1. Vorrichtung zur Fundusfotografie, umfassend:
eine Lichtquelle (1);
eine Abtasteinheit (13), die einen Spiegel enthält, der so konfiguriert ist, dass er biaxial in einer horizontalen Richtung und einer vertikalen Richtung angetrieben wird, um einen von der Lichtquelle emittierten Lichtstrahl (60) zweidimensional abzutasten;
ein optisches System (14), das so konfiguriert ist, dass es den Lichtstrahl, der von dem Spiegel der Abtasteinheit reflektiert wurde, in ein Auge (70) einer Person eintreten lässt; und
einen Lichtdetektor (40), der so konfiguriert ist, dass er den von einer Netzhaut (74) der Person reflektierten Lichtstrahl erfasst,
wobei ein Außendurchmesser des Spiegels der Abtasteinheit 1,7 mm oder mehr und 2,0 mm oder weniger beträgt, wobei der Spiegel der Abtasteinheit kreisförmig oder elliptisch ist, und wobei der Außendurchmesser ein Durchmesser des Spiegels der Abtasteinheit ist, wenn der Spiegel der Abtasteinheit kreisförmig ist, und ein kleinerer Durchmesser des Spiegels der Abtasteinheit ist, wenn der Spiegel der Abtasteinheit elliptisch ist,
wobei ein effektiver Durchmesser des Spiegels der Abtasteinheit 1,5 mm oder mehr und 1,8 mm oder weniger beträgt, wobei der effektive Durchmesser des Spiegels der Abtasteinheit gleich einem Wert ist, der durch Subtraktion von 0,2 mm von dem Außendurchmesser des Spiegels der Abtasteinheit erhalten wird, wobei die optische Vergrößerung des optischen Systems 0,8 mal oder mehr und 1,2 mal oder weniger beträgt,
wobei eine Resonanzfrequenz in der horizontalen Richtung der Abtasteinheit 6 kHz oder mehr und 12 kHz oder weniger beträgt.

2. Vorrichtung zur Fundusfotografie nach Anspruch 1, wobei ein mechanischer Ablenkungswinkel in mindestens einer der horizontalen Richtung oder der vertikalen Richtung der Abtasteinheit (13) 13 Grad oder mehr und 16 Grad oder weniger im halben Winkel beträgt.

3. Vorrichtung zur Fundusfotografie nach einem der Ansprüche 1 bis 2, wobei der Durchmesser des Lichtstrahls zum Zeitpunkt des Eintritts in das Auge (70) der Person 1,25 mm oder mehr beträgt und die Fleckgröße des Lichtstrahls auf der Netzhaut (74) 25 µm oder weniger beträgt.

4. Vorrichtung zur Fundusfotografie nach einem der Ansprüche 1 bis 3, ferner umfassend:
eine Signalverarbeitungseinheit (32), die so konfiguriert ist, dass sie ein Ausgangssignal von dem Lichtdetektor (40) verarbeitet; und
eine Bilderzeugungseinheit (33), die so konfiguriert ist, dass sie auf der Grundlage eines von der Signalverarbeitungseinheit verarbeiteten Signals ein Fundusbild der Person erzeugt.

5. Vorrichtung zur Fundusfotografie nach einem der Ansprüche 1 bis 4, ferner umfassend:
einen Einstellungsmechanismus (12), der eine Kollimationslinse (20) und eine Dioptrieneinstellungslinse (23) umfasst,
wobei die Abtasteinheit (13) den von der Lichtquelle (11) ausgesandten Lichtstrahl (60) zweidimensional abtastet, durch die Kollimationslinse (20) in im Wesentlichen paralleles Licht umgewandelt und dann durch die Dioptrieneinstelllinse (23) in konvergentes Licht umgewandelt wird,
wobei das optische System (14) eine Projektionslinse (25) enthält, die bewirkt, dass der vom Spiegel der Abtasteinheit reflektierte Lichtstrahl in das Auge (70) der Person eintritt, wobei der in das konvergente Licht umgewandelte Lichtstrahl durch den Spiegel der Abtasteinheit in verschiedene Richtungen reflektiert wird,
wobei eine Vielzahl der in den verschiedenen Richtungen reflektierten Lichtstrahlen in einer kristallinen Linse (72) der Person oder in der Nähe der kristallinen Linse durch die Projektionslinse (25) konvergiert werden und
wobei jeder der Vielzahl der in die verschiedenen Richtungen reflektierten Lichtstrahlen zwischen der Abtasteinheit und der Projektionslinse (25) gebündelt wird, dann gestreut wird, durch die Projektionslinse (25) in im Wesentlichen paralleles Licht umgewandelt wird und in das Auge der Person eintritt.

6. Vorrichtung zur Fundusfotografie nach einem der Ansprüche 1 bis 4, ferner umfassend:
einen Einstellungsmechanismus (12), der eine Kollimationslinse (20) umfasst,
wobei die Abtasteinheit (13) den von der Lichtquelle (11) ausgesandten und durch die Kollimationslinse (20) in im Wesentlichen paralleles Licht umgewandelten Lichtstrahl (60) zweidimensional abtastet,
wobei das optische System (14) eine erste Projektionslinse (27a) und eine zweite Projektionslinse (27b) umfasst,
wobei der durch die Kollimationslinse (20) in das im Wesentlichen parallele Licht umgewandelte Lichtstrahl durch den Spiegel der Abtasteinheit in verschiedene Richtungen reflektiert wird,
wobei eine Vielzahl der in den verschiedenen Richtungen reflektierten Lichtstrahlen durch die erste Projektionslinse (27a) parallel zueinander veranlasst werden, in die zweite Projektionslinse (27b) eintreten und durch die zweite Projektionslinse (27b) veranlasst werden, in einer Kristalllinse (72) der Person oder in der Nähe der Kristalllinse konvergiert zu werden, und
wobei jeder der Vielzahl der in die verschiedenen Richtungen reflektierten Lichtstrahlen durch die erste Projektionslinse (27a) in konvergentes Licht umgewandelt wird, zwischen der ersten Projektionslinse (27a) und der zweiten Projektionslinse (27b) gebündelt wird, dann gestreut wird, durch die zweite Projektionslinse (27b) in im Wesentlichen paralleles Licht umgewandelt wird und in das Auge der Person eintritt.

## Revendications

1. Dispositif de photographie de fond d'œil comprenant :
une source de lumière (1) ;
une unité de balayage (13) qui comporte un miroir configuré pour se déplacer de manière biaxiale dans une direction horizontale et une direction verticale pour balayer de manière bidimensionnelle un faisceau lumineux (60) émis par la source de lumière ;
un système optique (14) configuré pour amener le faisceau lumineux qui a été réfléchi par le miroir de l'unité de balayage à pénétrer dans un œil (70) d'un sujet ; et
un détecteur de lumière (40) configuré pour détecter le faisceau lumineux qui a été réfléchi par une rétine (74) du sujet,
dans lequel un diamètre externe du miroir de l'unité de balayage est de 1,7 mm ou plus et de 2,0 mm ou moins, dans lequel le miroir de l'unité de balayage est circulaire ou elliptique, et dans lequel le diamètre externe est un diamètre du miroir de l'unité de balayage lorsque le miroir de l'unité de balayage est circulaire, et est un diamètre mineur du miroir de l'unité de balayage lorsque le miroir de l'unité de balayage est elliptique,
dans lequel un diamètre effectif du miroir de l'unité de balayage est de 1,5 mm ou plus et de 1,8 mm ou moins dans lequel le diamètre effectif du miroir de l'unité de balayage est égal à une valeur obtenue en soustrayant 0,2 mm du diamètre externe du miroir de l'unité de balayage,
dans lequel un grossissement optique du système optique est de 0,8 fois ou plus et de 1,2 fois ou moins,
dans lequel une fréquence de résonance dans la direction horizontale de l'unité de balayage est de 6 kHz ou plus et de 12 kHz ou moins.

2. Dispositif de photographie de fond d'œil selon la revendication 1, dans lequel un angle de déviation mécanique dans au moins l'une de la direction horizontale ou de la direction verticale de l'unité de balayage (13) est de 13 degrés ou plus et de 16 degrés ou moins en demi-angle.

3. Dispositif de photographie de fond d'œil selon l'une quelconque des revendications 1 et 2, dans lequel un diamètre du faisceau lumineux au moment de la pénétration dans l'œil (70) du sujet est de 1,25 mm ou plus, et une taille de spot du faisceau lumineux sur la rétine (74) est de 25 µm ou moins.

4. Dispositif de photographie de fond d'œil selon l'une quelconque des revendications 1 à 3, comprenant également :
une unité de traitement de signal (32) configurée pour traiter un signal de sortie provenant du détecteur de lumière (40) ; et
une unité de génération d'image (33) configurée pour générer une image de fond d'œil du sujet, sur la base d'un signal traité par l'unité de traitement de signal.

5. Dispositif de photographie de fond d'œil selon l'une quelconque des revendications 1 à 4, comprenant également :
un mécanisme de réglage (12) qui comporte une lentille de collimation (20) et une lentille de réglage dioptrique (23),
dans lequel l'unité de balayage (13) balaie de manière bidimensionnelle le faisceau lumineux (60) qui a été émis par la source de lumière (11), converti en lumière sensiblement parallèle par la lentille de collimation (20) puis converti en lumière convergente par la lentille de réglage dioptrique (23), dans lequel le système optique (14) comporte une lentille de projection (25) qui amène le faisceau lumineux qui a été réfléchi par le miroir de l'unité de balayage à pénétrer dans l'œil (70) du sujet,
dans lequel le faisceau lumineux qui a été converti en lumière convergente est réfléchi dans différentes directions par le miroir de l'unité de balayage,
dans lequel une pluralité des faisceaux lumineux réfléchis dans les différentes directions sont convergés dans une lentille cristalline (72) du sujet ou à proximité de la lentille cristalline par la lentille de projection (25) et
dans lequel chacun de la pluralité des faisceaux lumineux réfléchis dans les différentes directions est condensé entre l'unité de balayage et la lentille de projection (25), est ensuite diffusé, est converti en lumière sensiblement parallèle par la lentille de projection (25), et pénètre dans l'œil du sujet.

6. Dispositif de photographie de fond d'œil selon l'une quelconque des revendications 1 à 4, comprenant également :
un mécanisme de réglage (12) qui comporte une lentille de collimation (20),
dans lequel l'unité de balayage (13) balaie de manière bidimensionnelle le faisceau lumineux (60) qui a été émis par la source lumineuse (11) et converti en lumière sensiblement parallèle par la lentille de collimation (20),
dans lequel le système optique (14) comporte une première lentille de projection (27a) et une seconde lentille de projection (27b),
dans lequel le faisceau lumineux qui a été converti en lumière sensiblement parallèle par la lentille de collimation (20) est réfléchi dans différentes directions par le miroir de l'unité de balayage,
dans lequel une pluralité des faisceaux lumineux réfléchis dans les différentes directions sont amenés à être parallèles les uns aux autres par la première lentille de projection (27a), pénètrent dans la seconde lentille de projection (27b) et sont amenés à converger dans une lentille cristalline (72) du sujet ou à proximité de la lentille cristalline par la seconde lentille de projection (27b), et
dans lequel chacun de la pluralité des faisceaux lumineux réfléchis dans les différentes directions est converti en lumière convergente par la première lentille de projection (27a), est condensé entre la première lentille de projection (27a) et la seconde lentille de projection (27b), est ensuite diffusé, est converti en lumière sensiblement parallèle par la seconde lentille de projection (27b), et pénètre dans l'œil du sujet.
